# EUROPEAN PATENT APPLICATION

(11) **EP 2 604 259 A1**
(43) Date of publication of application: **19.06.2013**
(21) Application number: 12189955.3
(22) Date of filing: 25.10.2012
(51) Int. Cl.: A61K 31/135, A61K 31/27, A61P 19/08, A61P 19/10

(54) **LSD-1 enzyme inhibitors for inducing osteogenic differentiation**

(30) Priority: 28.10.2011 IT MI20111971
(71) Applicant: Mesogenics S.r.l., 80121 Napoli (IT)
(72) Inventor: Salvatore, Francesco, 80121 NAPOLI (IT)
(74) Representative: Bertuccio, Silvia

(57) **Abstract**

The present invention relates to the use of LSD-1 enzyme inhibitors in the osteogenic differentiation of cells capable to differentiate into osteoblasts, especially mesenchymal stem cells, adult stem cells, and cells with osteogenic potential

## Description

### Field of invention

The present invention relates to the use of LSD-1 enzyme inhibitors in the osteogenic differentiation of cells capable of differentiating into osteoblasts.

### Prior art

LSD-1 (lysine-specific demethylase-1) is a gene that encodes for a flavin-dependant monoamine oxidase, which can demethylate mono- and di-methylated lysine, specifically histone 3, lysines 4 and 9 (H3K4 and H3K9).

LSD-1, discovered by Yujiang in the Harvard Medical School's Yang Shi laboratory, was the first lysine-demethylating enzyme to be discovered.

By means of a FAD-dependent oxidation reaction, LSD-1 specifically removes the methylation on histone H3 position K4 from me2 to me1 or me0.

When a complex forms with an androgen receptor (and other nuclear hormone receptors), LSD-1 goes back to H3K9me2.

The functions of other components of the complex containing LSD-1, such as CoREST and BHC80, have been further studied.

More recently, it was demonstrated that LSD-1 is also able to demethylate lysine residues on non-histone proteins like p53 Dnmt1 and E2F1.

Histone modification with enzymes is a key biological phenomenon in many differentiation processes.

LSD-1 has been identified as the key histone modifier involved in maintaining the pluripotency essential to maintain the right balance between H3K4me2/me3 and H3K27me3, which are important in regulating stemness. High levels of LSD-1 are needed to maintain the undifferentiated state of human embryo stem cells by means of strict control of the H3K4 methylation levels in the regulation regions of the differentiation genes. Thus the "down-regulation" or inhibition of LSD-1 during differentiation can constitute an essential differentiation-guiding mechanism. In practice, changes in expression levels or inhibition of LSD-1 can control the balance between self-renewal and maturity.

### Summary of the invention

The present invention relates to LSD-1 enzyme inhibitors for use in the osteogenic differentiation of cells capable of differentiating into osteoblasts, and in the maintenance of bone maturation.

### Detailed description of the invention

It has surprisingly been found that inhibition of the enzyme LSD-1 (lysine-specific demethylase-1) induces osteogenic differentiation, *in vitro* and *in vivo,* of cells with the potential to differentiate into osteoblasts, such as primary human mesenchymal stem cells obtained from bone marrow or primary mesenchymal stem cells derived from mice, or into cell lines derived from the latter, such as type W20-17.

Inhibition of enzyme LSD-1 also induces maintenance of bone maturation.

The present invention relates to the use of LSD-1 enzyme inhibitors in the osteogenic differentiation of cells capable of differentiating into osteoblasts.

The invention relates to the use of LSD-1 enzyme inhibitors to maintain bone maturation.

According to a preferred aspect of the invention, cells capable of differentiating into osteoblasts are mesenchymal stem cells, adult stem cells (ASC), and cells with osteogenic potential.

Osteoblast differentiation consists of transformation, following appropriate biological, physical and/or pharmacological stimuli, of cells such as ASCs deriving from various tissues such as bone marrow, adipose tissue, connective tissue, muscle, into osteoblasts, or into cells capable of producing mineralised bone matrix. Induction of this biological phenomenon has a number of applications in the field of laboratory activities for the study of the cell changes connected with this process, and in the therapeutic field for disorders characterised by mineralised bone tissue deficiency such as osteoporosis, osteomalacia and discontinuous fractures.

According to the present invention, compounds able to inhibit enzyme LSD-1 are preferably tranylcypromine and compound MC2580 of formula (I):

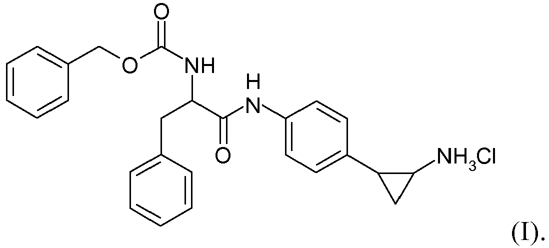

Preferably, tranylcypromine and MC2580 are capable of inducing osteogenic differentiation of adult stem cells into osteoblasts.

LSD-1 enzyme inhibitors can be used for therapeutic purposes in the orthopaedic and surgical fields.

According to a preferred aspect, LSD-1 enzyme inhibitors can be used in patients suffering from bone disorders due, for example, to reduced new bone synthesis or increased bone resorption, such as osteoporosis, osteomalacia and discontinuous bone fractures, and in patients suffering from disorders which can benefit from inducing osteogenic differentiation.

LSD-1 inhibitors can also be used for therapeutic purposes for arthrodesis, medical devices able to induce bone synthesis to repair fractures, or any use in the orthopaedic and/or surgical fields.

LSD-1 enzyme inhibition can be used to induce osteogenic differentiation in cell cultures by using LSD-1 enzyme inhibitors in the preparation of culture media, serum substitutes and/or culture additives.

LSD-1 enzyme inhibitors can be used alone or in combination with other suitable active ingredients.

The examples given below further illustrate the invention.

### Examples

### Example 1 - LSD-1 enzyme inhibition and increased osteogenic differentiation

W20-17 cells or primary mesenchymal stem cells seeded on 96-well or 6-well plates or any other type of culture medium, if grown with an osteogenesis-inducing medium (D-MEM supplemented with 10% foetal bovine serum, 4 mM L-glutamine, 0.05 mM ascorbic acid 2-phosphate, 10 mM glycerol 2-phosphate and 10⁻⁶ M dexamethasone), differentiate into osteoblasts in 14 days, and produce mineralised bone matrix in 21 days. Differentiation into osteoblasts is evaluated by testing for the appearance of the enzyme alkaline phosphatase, the activity of which is identified by histochemical staining. The presence of mineralised matrix is evaluated by histochemical analysis with Alizarin Red staining, which reveals the mineralised precipitates.

W20-17 cells were seeded in 96-well plates at a concentration of 6000 cells x cm² and grown in osteogenic medium, either alone or supplemented with medicaments able to inhibit the activity of LSD-1, tranylcypromine and MC2580 (50 µM).

On the 14th day, staining was performed to evaluate the activity of alkaline phosphatase, a marker expressed by osteoblasts during differentiation.

After one wash with PBS, the cells were fixed with a 10% solution of neutral formalin buffer (NFB) and incubated for 45 minutes at ambient temperature with a solution containing 0.24 mM naphthol AS MX-PO4, 0.4% N,N-dimethylformamide and red violet LB salt in 0.2 M Tris-HCl at pH 8.3.

After washing with distilled water, the cells that tested positive for alkaline phosphatase expression were observed under the microscope.

The results demonstrated that LSD-1 enzyme inhibition increases osteogenic differentiation, because the administration of both the LSD-1 inhibitors induces greater expression of alkaline phosphatase and a deeper stain than treatment with osteogenic medium only.

### Example 2 - LSD-1 enzyme inhibition and increased mineralisation

This test was conducted to evaluate whether LSD-1 inhibition also increases the deposit of mineralised matrix, which distinguishes the last stage of osteogenic differentiation.

W20-17 cells were seeded in 96-well plates and maintained in culture with osteogenic medium, either alone or supplemented with compound MC2580.

On the 21 st day, the presence of mineralised matrix was evaluated by histochemical staining with Alizarin Red. The cells were washed with PBS, fixed with a 10% formaldehyde solution, and incubated with a 2% solution of Alizarin Red (pH 4.1-4.3) for one hour.

After several washes with distilled water, the plate was observed under the microscope to evaluate the extent of mineralisation. For a more precise test, after observation under the microscope, the stain was quantitated by extracting the stain bonded to the calcium deposits with guanidine-HCl 4 M, and determining the absorbance at 490 nm.

The results demonstrated that LSD-1 enzyme inhibition with pharmacological treatment increases the deposit of mineralised matrix compared with treatment with osteogenic medium alone.

The effect of LSD-1 inhibition on osteogenic differentiation and mineralisation was also evaluated by von Kossa staining, which identifies the presence of calcium salts.

At the end of the treatment with osteogenic medium or osteogenic medium supplemented with compound MC2580, the W20-17 cells were washed with PBS, fixed with a 10% formaldehyde solution and incubated with a 5% silver nitrate solution for 40 minutes under a UV lamp. After exposure to ultraviolet light the silver nitrate was reduced, and the metallic silver fixed instead of the calcium. The excess silver was removed with a 5% solution of sodium thiosulphate, and after washes with distilled water, the calcium or calcium salt deposits can be observed under the microscope.

Once again, the results demonstrate that treatment with LSD-1 inhibitors induces an increase in mineralisation compared with treatment with osteogenic medium alone.

### Example 3 - Expression profiles of osteogenic markers

The different stages of osteogenic differentiation are characterised by the expression of a series of osteogenic markers, including transcription factors RUNX-2 and Osterix, the sialoprotein Osteopontin, and Osteocalcin.

The effect of two LSD-1 inhibiting compounds, tranylcypromine and MC2580, was evaluated on the expression profile of three osteogenic markers (osteopontin, osterix and osteocalcin) in W20-17 cells cultured with osteogenic medium.

After 21 days' treatment, the total RNA was extracted with TriReagent, and cDNA reverse transcription was performed. The reverse transcription (RT-PCR, reverse transcription polymerase chain reaction) was conducted with 2 µg of total RNA, 5 µM random primers and reverse transcriptase M-MuLV. The expression levels of the genes of interest were subsequently determined by real-time PCR, using SYBR Green PCR master mixture and an ABI PRISM 7500 apparatus (Applera, Foster City, CA).

The expression levels of the target genes were quantified with primers specific for the gene of interest and normalised on the basis of expression of glyceraldehyde-3-phosphate dehydrogenase (GAPDH). The results demonstrate that, unlike W20-17 cells cultured with osteogenic medium alone, treatment with LSD-1 inhibiting compounds induces a significant increase in expression of the genes osteopontin and osterix, and a reduction in the expression of late marker osteocalcin, which is most marked in the case of administration of compound MC2580.

### Example 4 - LSD-1 enzyme inhibition and promotion of osteogenic differentiation in the absence of other stimuli

To establish whether LSD-1 enzyme inhibition is sufficient to induce differentiation into osteoblasts, this assay was performed to evaluate the activity of alkaline phosphatase on W20-17 cells cultured with normal medium (D-MEM), or D-MEM supplemented with tranylcypromine or MC2580.

The results demonstrate that treatment with LSD-1 inhibiting compounds induces a significant increase in the activity of alkaline phosphatase compared with the negative control (cells cultured with D-MEM alone), thus indicating that inhibition of LSD-1 is capable of inducing osteogenic differentiation in the absence of other stimuli.

### Example 5 - LSD-1 enzyme inhibition and promotion of mineralisation in the absence of other stimuli

To evaluate the minimum composition of a medium capable of inducing the formation of mineralised matrix, W20-17 cells were seeded in a 96-well plate and cultured for 21 days under different growth conditions.

At the end of the treatment the cells were stained with Alizarin Red, as described in example 2, to evaluate the formation of mineralised nodules.

The staining results indicate that the inhibition of LSD-1 enzyme activity obtained by administering tranylcypromine or compound MC2580 promotes mineralisation, even in the absence of the osteogenesis inducers dexamethasone and ascorbic acid.

However, the administration of glycerol 2-phosphate is necessary, because that factor acts as donor of the phosphate groups essential for the formation of mineralised nodules.

### Example 6 - The activity of enzyme LSD-1 is inhibited during osteogenic differentiation

To evaluate whether the activity of enzyme LSD-1 is inhibited during osteogenic differentiation, an *in vitro* enzymatic activity assay was performed on W20-17 cells to assess the percentage inhibition of LSD-1 activity under different conditions.

The results demonstrate that the activity of the enzyme is inhibited during osteogenic differentiation (cells cultured with osteogenic medium) and that the percentage inhibition increases when the culture medium is supplemented with compound MC2580.

### Example 7 - Expression of enzyme LSD-1 is low during the initial stages of differentiation

The expression profile of gene LSD-1 was evaluated in W20-17 cells during osteogenic differentiation.

The total RNA was extracted after 0, 4, 14 and 21 days of differentiation, and the expression level of LSD-1 was determined by real-time PCR, according to the procedure described in example 3.

The results demonstrate that the gene of interest is expressed at very low levels during the initial stages of differentiation (t0 and t4), but its expression starts to increase from the 14th day, suggesting that LSD-1 may be down-regulated during the early stages of osteogenesis.

## Claims

1. LSD-1 enzyme inhibitors for use in the osteogenic differentiation of cells capable of differentiating into osteoblasts and/or in the maintenance of bone maturation.

2. Inhibitors for use according to claim 1, wherein the cells are mesenchymal stem cells.

3. Inhibitors for use according to claim 1, wherein the cells are adult stem cells.

4. Inhibitors for use according to claim 1, wherein the cells are cells with osteogenic potential.

5. Inhibitors for use according to claims 1-4, wherein the inhibitor is tranylcypromine.

6. Inhibitors for use according to claims 1-4, wherein the inhibitor is the compound MC2580 of formula (I):

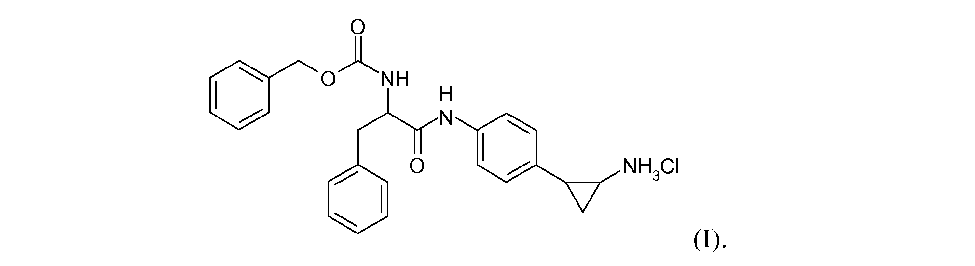

7. Inhibitors for use according to claims 1-6, in the surgical and/or orthopaedic field.

8. Inhibitors for use according to claims 1-7, in the treatment of bone diseases due to a reduction of the bone neo-synthesis or to the increase of the bone resorption.

9. Inhibitors for use according to claim 8, wherein the bone diseases are osteoporosis, osteomalacia and discontinuous bone fractures.

10. Inhibitors for use according to claims 1-7, in arthrodesis and/or medical devices for use in bone synthesis induction for fracture repair.

11. Inhibitors for use according to claims 1-6, in the preparation of culture media and/or serum substitutes and/or any additive for the cell culture.

12. Pharmaceutical composition comprising an LSD-1 enzyme inhibitor for use in the osteogenic differentiation of cells capable of differentiating into osteoblasts and/or in the maintenance of bone maturation and a pharmaceuitically acceptable adjuvant or carrier.
